# EUROPEAN PATENT APPLICATION

(11) **EP 3 437 697 A1**
(43) Date of publication of application: **06.02.2019**
(21) Application number: 18186474.5
(22) Date of filing: 31.07.2018
(51) Int. Cl.: A61Q 1/10, A61K 8/92, A61K 8/97

(54) **BAMBOO FIBERS IN A HIGH-DEFINITION NATURAL-BASED MASCARA FOR EYELASHES AND EYEBROWS**

(30) Priority: 04.08.2017 IT 201700090879
(71) Applicant: Virgilio Holding S.p.A., 20138 Milano (IT)
(72) Inventor: Anselmi, Cecilia, 53100 Siena (IT); Centini, Marisanna, 53027 San Quirico d'Orcia (SI) (IT); Silverio, Cristina, 84034 Padula (SA) (IT); Tola, Maria Francesca, 90047 Partinico (PA) (IT)
(74) Representative: Gerli, Paolo

(57) **Abstract**

New composition of a natural-based mascara, comprising bamboo fibers, free from synthetic fibers, having optimal features in terms of volumization and adhesiveness to eyelashes and eyebrows.

## Description

### FIELD OF THE INVENTION

The present invention is set in the field of research and development of innovative bio and eco-sustainable natural-based cosmetic products, which are highly performing and safe. New compositions for make-up of eyes, in particular eyelashes and eyebrows, comprising natural fibers associated with emulsifiers and other ingredients known in the field are described. The resulting products have a high volumizing and thickening effect on eyelashes and eyebrows, avoiding the commonly used synthetic film-forming polymers.

### PRIOR ART

Mascara is a cosmetic product for application on eyelashes and eyebrows: the main cosmetic effects required for this kind of product are the increase in volume (volumization), lengthening and curvature of eyelashes, definition, volume and thickening effect of eyebrows; furthermore, mascaras must have useful features for their application, namely sufficient fluidity, spreadability, adhesiveness to eyelashes, absence of lumps, easy removability during make-up removal, etc.; these features are not always easy to reconcile with the above cosmetic effects.

Mascaras are generally based on emulsifying systems associated with oils, waxes or lipophilic substances, in order to obtain a sufficiently fluid base; polymeric and/or fibrous products, useful to give consistency and obtain the thickening/lengthening of eyelashes and definition of eyebrows, in addition to dyes to give colour, are associated therewith; the use of film-forming agents favouring product adhesion and uniform coating of eyelashes and eyebrows is also widespread.

With particular reference to eyebrows, delineators for eyebrows in water resistant cream, which fill in, outline and shape eyebrows thus ensuring a long-lasting tightness, are known in the field.

Mascara mixtures enriched with volumizing fibers, applicable by a brush on eyelashes or eyebrows. have been recently introduced into the field.

For instance, the commercial product "eyebrown fibers coloured mascara" distributed by Kiko consists of a mascara for eyebrows comprising synthetic polymers, in particular nylon.

Several are the publications and patents in the field, often focused on specific ingredients of the mascara and/or combinations thereof.

For instance, KR2000026081 discloses a mascara comprising vegetable fibers among which hemp powder and bamboo, and synthetic polymers, specifically alkyl acrylate, to obtain a volumizing effect.

In this field the use of synthetic film-forming polymers, such as e.g. polyacrylates, polyurethanes, polyamides, polyolefins, silicones and polymers thereof, polyesters, polyacetates, etc., is very frequent; said products are less and less desired by the consumer, both for the possibility of incorporating toxic residues resulting from the synthesis process, and for the growing tendency to prefer natural-based products having low-impact on the body and environment.

However, it is difficult to avoid the use of these products since they are essential to promote a high adhesion and homogeneous coating of eyelashes and eyebrows, so that the tendency to reduce the "synthetic" component of mascara is often accompanied by a reduction in performance, e.g. in terms of coating adhesiveness or homogeneity. The present invention faces and solves these problems by providing a new "natural-based" mascara, having a simple and effective composition and avoiding the use of synthetic polymers, having performance features which are equal to or even greater than those of the commonly used products.

### SUMMARY

The object of the present invention is a new composition for the make-up of eyes, specifically for mascara, comprising bamboo fibers. The composition does not require the use of synthetic polymers and synthetic fibers and has optimal features in terms of volumization, lengthening and adhesiveness to eyelashes, and of definition and filling of eyebrows.

### DESCRIPTION OF THE FIGURES

Figure 1: comparison of artificial eyelashes on which a control mascara composition (blank, according to Example 3), a mascara composition obtained according to Example 1 and a mascara composition obtained according to Example 2 have been applied.
Figure 2: comparison of the definition effect of eyebrows obtained by a control mascara composition (blank, according to Example 7), a commercial mascara composition and a mascara composition obtained according to Example 5, in the presence or absence of a fixative.

### DETAILED DESCRIPTION OF THE INVENTION

The main object of the invention is a natural-based mascara comprising fibers, where said fibers consist of bamboo fibers.

In the present disclosure "mascara" means, as commonly known in the field, a product suitable for being applied on eyelashes and eyebrows and for the temporary coating thereof, comprising ingredients characteristic of mascara, such as emulsifiers, fibers, oils, waxes and dyes.

The term "natural-based" identifies a mascara in which the use of natural substances is preferred, in particular thanks to the exclusive use of vegetable fibers and avoiding the use of synthetic polymers; as for the other ingredients, the term "natural-based" allows the use of natural substances, but does not exclude the use of non-natural substances, e.g. synthetic or semi-synthetic substances.

As above indicated, in the mascara object of the invention the fiber component exclusively comprises vegetable fibers, specifically bamboo fibers.

The use of artificial fibers is not provided. The set of said bamboo fibers is generally comprised between 1 and 10% by weight on the final mascara, preferably between 3% and 10%, even more preferably 7%.

Said fibers preferably have length comprised between 10 µm and 120 µm, more preferably between 15 µm and 115 µm. Preferably said fibers have, within said range, a wide distribution of lengths. In fact, it has been observed that the dimensional heterogeneity of the bamboo fibers has further increased the volumizing, adhesive and lengthening effects of the composition. In particular, it turned out to be advantageous that at least 50% by weight of the bamboo fibers have length comprised between 60 and 115 µm.

The bamboo fibers used according to the present invention allow obtaining both a volumizing and a filling effect, thus turning out to be particularly suitable for the application on eyelashes and eyebrows.

In addition to the bamboo fibers, the present composition contains ingredients that are commonly requested in the mascara field: by non-limiting example, the following classes can be cited: emulsifiers, waxes, oils, dyes, preservatives, antioxidants generally in an aqueous vehicle; the film-forming agents are not necessary, since this function is effectively performed by the mixture of used fibers; the composition can thus be free from said agents.

The emulsifiers are generally used in a weight percentage on the weight of the mascara comprised between 1 and 10% and may be widely selected among those commercially available. Non-limiting examples are: potassium cetyl phosphate or glyceryl stearate citrate; other possible emulsifiers are those comprising starch, e.g. the product Emulprogress EC2 distributed by Prodotti Gianni S.p.A.

The oils are generally used in a weight percentage on the weight of the mascara comprised between 2 and 20%, and can be widely selected among those commercially available; examples of oils are: triglycerides of caprylic/capric acid, tricapriline, isoamyl laurate, vegetable oils such as e.g.: castor, sesame, flax, coconut, corn, cotton seeds, olive, palm, illipé, rape, soybean, sunflower, walnut, avocado, camellia, macadamia nut, grape seeds, peanut, jojoba, etc.

In an embodiment particularly suitable for application on eyelashes, castor oil is present in a weight percentage with respect to the weight of the mascara comprised between 1.5%-3.5%, whereas isoamyl laurate is present in a weight percentage with respect to the weight of the mascara comprised between 2.0%-3.0%.

In an embodiment particularly suitable for application on eyebrows, castor oil is present in a weight percentage with respect to the weight of the mascara comprised between 0.3%-0.7%, whereas isoamyl laurate is present in a weight percentage with respect to the weight of the mascara comprised between 3.0%-4.0%.

Thanks to these different proportions of the above oils, an optimized product for application on eyelashes and eyebrows is obtained.

Specifically, the mascara composition as above defined for application on eyebrows displays a good spread and a surprising volumizing effect, due to the effective adhesion of the bamboo fibers to eyebrows and to the presence of isoamyl laurate in the above percentages, which, being volatile, leaves on eyebrows a product that is drier and more durable over time. The above proportions may be varied according to the chemical-physical properties of the utilized oil.

In order to increase said dry effect while keeping a good volumizing and filling effect, the mascara composition may further contain, in a weight percentage on the weight of the mascara, 2.5%-3.5% of mica or magnesium stearate.

Waxes are generally used in a weight percentage on the weight of the mascara comprised between 3 and 25% and may be widely selected among those commercially available. Non-limiting examples are: beeswax, spermaceti, lanolin, shellac wax, carnauba wax, candelilla wax, sugar cane wax, rice wax, olive wax. Among the favourite ones, carnauba wax and beeswax are to be mentioned.

Dyes can be preferably selected among mineral or organic pigments. The pigments may be white or coloured, possibly pearly. Among the mineral pigments, titanium dioxide, coated titanium dioxide, iron oxides, chromium oxides, manganese violet, ultramarine blue, etc. Among the organic pigments, carbon black and those permitted by Regulation (EC) 1223/2009 for the ocular zone. Examples of white pearly pigments is mica covered with titanium dioxide or bismuth oxychloride. Mica with titanium dioxide, iron oxides, etc. are examples of coloured pigment. Dyes are generally used in a weight percentage on the weight of the mascara comprised between 1 and 10%.

Preservatives and antioxidants, according to the invention, may be selected among those available and accepted in products with a "natural" connotation, generally used in a weight percentage which is variable according to known quantities as a function of the specific preservative. Benzoic acid, sorbic acid, dehydroacetic, benzyl alcohol, anisic acid, tocopherol and derivatives, tocopherol-based mixtures, etc. are non-limiting examples of preservatives and antioxidants.

Further possible ingredients are, without limitation: consistency factors, in proportions preferably comprised between 1 and 10% by weight on the mascara (e.g. glyceryl behenate, glyceryl stearate), additives with "caring" effect, perfumes, emollients, sequestrants, neutralizing agents, sunscreens, lacquers, fillers, rheological modifiers, stabilizers, pH correctors, humectants (e.g. glycerin), moisturizers, vitamins, hyaluronic acid, etc.

The carrier is an aqueous solvent, preferably water, typically in the emulsified phase, preferably oil-in-water, with the above cited components.

In particular, in the embodiment in which the composition of mascara of the present invention is applied on eyelashes, the carrier is an oil-in-water emulsion.

In fact, said carrier allows obtaining a volumizing and filling effect to eyelashes, since the first applications (Examples 1 and 2).

An important feature of the mascaras of the present invention is the absence of any polymer of synthetic nature, (said ingredients are widely used in mascaras, in particular as film-forming agents); in fact, the bamboo fibers, in combination with the above cited components, give the mascara excellent features of adhesiveness and coating homogeneity, so as to make the use of such agents superfluous; advantageously, the absence of synthetic polymers increases the pleasantness of the product and, especially, avoids exposing the user to traces of by-products deriving from polymerization processes; thus a "safer" cosmetic product is obtained, having less impact on the user and environment. The synthetic polymers absent from the mascaras of the invention are typically, but not exclusively, those used as filmogens and are represented, without limitation, by: polymers or acrylic copolymers (e.g. acrylates, methacrylates, alkyl acrylates, alkyl methacrylates, polyacrylates, polymethacrylates), polyolefins, polyvinyls, polyurethanes, polyamides, polyimides, polyethers, polyesters, fluoropolymers, polyethers, polyacetates, polycarbonates, rubbers, epoxides, aldehyde resins, polysiloxanes, polyquaterniums and the like.

A further important feature of the mascaras of the present invention, consequent to the above described selection of fibers, is the absence of any synthetic fiber, e.g. nylon, rayon, etc. In fact, the bamboo fibers, in combination with the above cited compounds, turned out to be suitable for obtaining the herein described effects, without using synthetic fibers.

The invention further comprises the use of a mascara as above described in cosmetics, namely as a product for eyes, in particular for covering eyelashes and/or eyebrows with a make-up effect, but also for protection since it is free from synthetic polymers and synthetic fibers. The use of the natural-fiber mixture as above described allows optimally obtaining the effects required in a mascara: increasing volume and length of eyelashes with an effect free from smudges and lumps, with a good separation among eyelashes and an excellent curvature of eyelashes. The lengthening and volumizing effect tuned out to be comparable to or greater than the one obtainable by mascaras containing polymers and synthetic fibers. In particular, as shown in the examples, a composition according to the invention comprising fibers and bamboo improves separation, length, thickness, resistance to spreading. Moreover, the mascaras of the invention are easy to remove and do not damage eyelashes.

Moreover, said mascara composition may be applied on eyelashes, giving a volumizing and flaming effect, and are able to colour eyebrows, thanks to the presence of pigments in the mascara of the invention, without however painting or staining the skin.

An even more evident fixative effect can be obtained by applying the mascara composition according to the present invention and, successively, a fixative-action gel formulated with 2% hyaluronic acid. The combined application of said products involves a safe protection of eyebrows, in addition to a greater fixing and a greater durability over time of the mascara initially applied, without undesired release and/or smudges of the product.

### EXPERIMENTAL EXAMPLES

In the non-limiting examples herein below reported mascara formulations according to the invention are shown, unless otherwise indicated. Furthermore, the effect obtained by application on standard artificial eyelashes and on eyebrows is shown. All of the compositions according to the invention have displayed evident abilities of eyelash lengthening and volumization, of eyebrows definition, coloring and thickening and with an evident film-forming effect. The quantities shown in the examples are expressed in grams.

According to example 1, the emulsifier is Emulprogress EC2. The wax mixture consists of beeswax and carnauba wax. Consistency factors are glyceryl monostearate and glyceryl behenate. Vegetable oils are castor oil and triglyceride of caprylic/capric acid. The pigment is titanium dioxide, plus coloured pigments. Tocopherol, benzyl alcohol, ethylhexylglycerin constitute the preservative and antioxidant system. The bamboo fibers are present by 7%.

Examples 1 and 2 refer to compositions for mascara to be applied on eyelashes, examples 3, 4 refer to reference compositions (blank); examples 5 and 6 refer to compositions to be applied on eyebrows, example 7 refers to the reference composition (blank).

| **EXAMPLE 1** | |
|---|---|
| *Emulprogress EC2 (emulsifier)* | 3 |
| *Glyceryl Stearate* | 1.5 |
| *Cerferous copemicia (Carnauba) Wax* | 3 |
| *Beeswax Alba Wax* | 6 |
| *C12-15 Alkyl Benzoate* | 6.5 |
| *Ricinus communis seed oil* | 2 |
| *Cyclopentasiloxane* | 2.5 |
| *Dimethicone* | 1 |
| *Glycerin* | 4 |
| *Water* | q. s. to 100 |
| *Bamboo fibers* | 7 |
| *Pigments (white and coloured)* | 5.5 |
| *Benzyl Alcohol, Ethylhexylglycerin, Tocopherol* | 1 |
| *Tocopherol* | 1 |
| *Glyceryl behenate* | 3 |

| **EXAMPLE 2** | |
|---|---|
| *Emulprogress EC2 (emulsifier)* | 3 |
| *Glyceryl Stearate* | 1.5 |
| *Cerferous copernicia (Carnauba) Wax* | 3 |
| *Beeswax Alba Wax* | 6 |
| *Caprylic*/*Capric Triglycerides* | 6.5 |
| *Ricinus communis seed oil* | 3 |
| *Isoamyl laurate* | 2.5 |
| *Glycerin* | 4 |
| *Water* | q. s. to 100 |
| *Bamboo fibers* | 7 |
| *Pigments (white and coloured)* | 5.5 |
| *Benzyl Alcohol, Ethylhexylglycerin, Tocopherol* | 1 |
| *Tocopherol* | 1 |
| *Glyceryl behenate* | 3 |

| **EXAMPLE 3 (reference)** | |
|---|---|
| *Emulprogress EC2 (emulsifier)* | 3 |
| *Glyceryl Stearate* | 1.5 |
| *Cerferous copemicia (Camauba) Wax* | 3 |
| *Beeswax Alba Wax* | 6 |
| *C12-15 Alkyl Benzoate* | 6.5 |
| *Ricinus communis seed oil* | 2 |
| *Cyclopentasiloxane* | 2.5 |
| *Dimethicone* | 1 |
| *Glycerin* | 4 |
| *Water* | q. s. to 100 |
| *Pigments (white and coloured)* | 5.5 |
| *Benzyl Alcohol, Ethylhexylglycerin, Tocopherol* | 1 |
| *Tocopherol* | 1 |
| *Glyceryl behenate* | 3 |

| **EXAMPLE 4 (reference)** | |
|---|---|
| *Emulprogress EC2 (emulsifier)* | 3 |
| *Glyceryl Stearate* | 1.5 |
| *Cerferous copemicia (Camauba) Wax* | 3 |
| *Beeswax Alba Wax* | 6 |
| *Caprylic*/*Capric Triglycerides* | 6.5 |
| *Ricinus communis seed oil* | 3 |
| *Isoamyl laurate* | 2.5 |
| *Glycerin* | 4 |
| *Water* | q. s. to 100 |
| *Pigments (white and coloured)* | 5.5 |
| *Benzyl Alcohol, Ethylhexylglycerin, Tocopherol* | 1 |
| *Tocopherol* | 1 |
| *Glyceryl behenate* | 3 |

| **EXAMPLE 5** | |
|---|---|
| *Potassium Cetyl Phosphate* | 1.5 |
| *Cellulolose Gum, Carrageenan, Ceratonia Siliqua Gum, Sucrose* | 1 |
| *Glyceryl Stearate* | 1.5 |
| *Cerferous copernicia (Carnauba) Wax* | 3 |
| *Beeswax Alba Wax* | 6 |
| *Caprylic*/*Capric Triglycerides* | 3 |
| *Ricinus communis seed oil* | 0.5 |
| *Isoamyl laurate* | 3.5 |
| *Glycerin* | 4 |
| *Water* | q. s. to 100 |
| *Bamboo fibers* | 7 |
| *Pigments (white and coloured)* | 5.5 |
| *Benzyl Alcohol, Ethylhexylglycerin, Tocopherol* | 1 |
| *Tocopherol* | 1 |
| *Glyceryl behenate* | 3 |
| *Mica* | 3 |

| **EXAMPLE 6** | |
|---|---|
| *Potassium Cetyl Phosphate* | 1.5 |
| *Cellulolose Gum, Carrageenan, Ceratonia Siliqua Gum, Sucrose* | 1 |
| *Glyceryl Stearate* | 1.5 |
| *Cerferous copemicia (Carnauba) Wax* | 3 |
| *Beeswax Alba Wax* | 6 |
| *Caprylic*/*Capric Triglycerides* | 3 |
| *Ricinus communis seed oil* | 0.5 |
| *Isoamyl laurate* | 3.5 |
| *Glycerin* | 4 |
| *Water* | q. s. to 100 |
| *Bamboo fibers* | 7 |
| *Pigments (white and coloured)* | 5.5 |
| *Benzyl Alcohol, Ethylhexylglycerin, Tocopherol* | 1 |
| *Tocopherol* | 1 |
| *Glyceryl behenate* | 3 |
| *Magnesium stereate* | 3 |

| **EXAMPLE 7 (reference)** | |
|---|---|
| *Potassium Cetyl Phosphate* | 1.5 |
| *Cellulolose Gum, Carrageenan, Ceratonia Siliqua Gum, Sucrose* | 1 |
| *Glyceryl Stearate* | 1.5 |
| *Cerferous copemicia (Carnauba) Wax* | 3 |
| *Beeswax Alba Wax* | 6 |
| *Caprylic*/*Capric Triglycerides* | 3 |
| *Ricinus communis seed oil* | 0.5 |
| *Isoamyl laurate* | 3.5 |
| *Glycerin* | 4 |
| *Water* | q. s. to 100 |
| *Pigments (white and coloured)* | 5.5 |
| *Benzyl Alcohol, Ethylhexylglycerin, Tocopherol* | 1 |
| *Tocopherol* | 1 |
| *Glyceryl behenate* | 3 |
| *Mica* | 3 |

According to reference examples 3, 4 and 7, the base is, as reported, fiberless.

An example of fixative, applicable on eyelashes/eyebrows after deposition of the concerned mascara, is given in the following example.

| **EXAMPLE 8** | |
|---|---|
| *Water* | q. s. to 100 |
| *Hyaluronic acid* | 2 |
| *Benzyl alcohol, Ethylhexylglycerin, Tocopherol* | 1 |

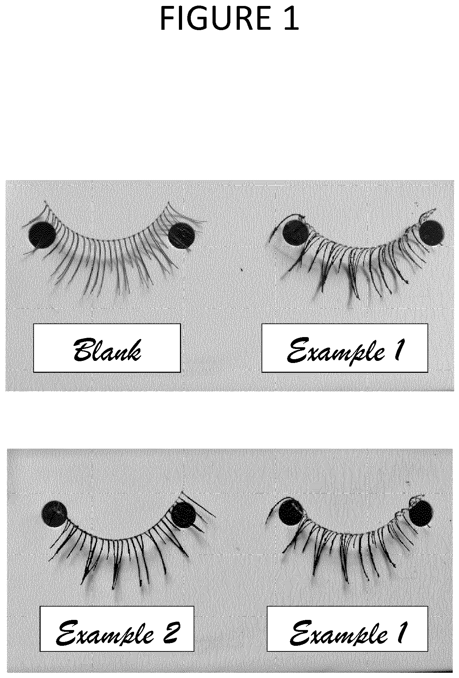

As shown in figure 1, the product prepared according to examples 1 and 2 turned out to be easily applicable on the artificial eyelashes and has the following effects: volumized, thick and longer eyelashes, without lumps, it does not spread out.

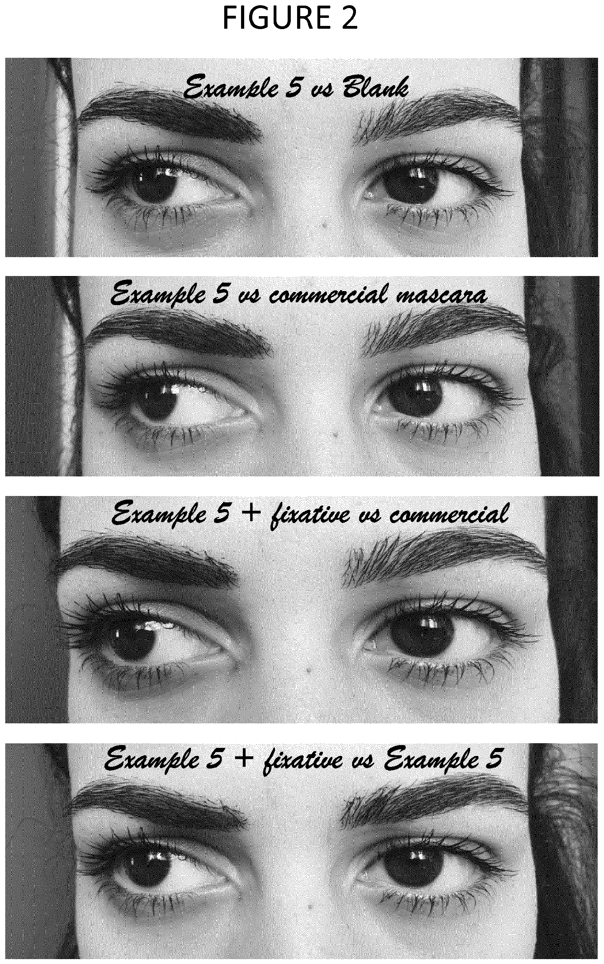

As evident in figure 2, the product prepared according to example 5 turned out to be easily applicable on eyebrows and has the following effects: volumized, thickened, defined and colored eyebrows, without staining the skin.

In summary, in all of the above reported examples of the invention, the product turns out to be easily spread on eyelashes and eyebrows, showing a surprising volumizing effect, due to the presence of the bamboo fibers in the mascara composition according to the invention.

Furthermore, the reported compositions do not damage, but protect, eyelashes and eyebrows are easily removed.

## Claims

1. Natural-based mascara comprising fibers, wherein said fibers consist of bamboo fibers and said mascara is free from synthetic polymers.

2. Mascara according to claim 1, wherein said bamboo fibers are present in a weight percentage from 1 to 10% on the weight of the mascara.

3. Mascara according to claims 1-2, wherein said bamboo fibers are present in a weight percentage from 3% to 10% on the weight of the mascara.

4. Mascara according to claims 1-3, wherein said bamboo fibers have average length from 10 to 120 µm.

5. Mascara according to claims 1-4, wherein said bamboo fibers have average length from 15 to 115 µm.

6. Mascara according to claims 1-5, wherein at least 50% of said bamboo fibers have length from 60 to 115 µm.

7. Mascara according to claims 1-6, comprising emulsifiers, oils, waxes, dyes.

8. Mascara according to claims 1-7 comprising, by weight on the weight of the mascara: 1-10% emulsifiers, 3-25% waxes, 2-20% oils, 1-10% dyes.

9. Mascara according to claims 1-8, wherein said oils comprise, in weight on the weight of the mascara, 1.5-3.5% castor oil and 2.0-3.0% isoamyl laurate, being said mascara to be applied on eyelashes.

10. Mascara according to claims 1-9, comprised in an oil-in-water emulsion, being said mascara to be applied on eyelashes.

11. Mascara according to claims 1-8, wherein said oils comprise, in weight on the weight of the mascara, 0.3-0.7% castor oil and 3.0-4.0% isoamyl laurate, being said mascara to be applied on eyebrows.

12. Mascara according to claim 11, further comprising, in weight on the weight of the mascara, 2.5-3.5% mica.

13. Mascara according to claim 11, further comprising, in weight on the weight of the mascara, 2.5-3.5% magnesium stearate.

14. Use of the mascara described in the claims 1-12 in cosmetics.
